(19) Europäisches Patentamt
European Patent Office
Office européen des brevets

(11) Publication number: 0 482 657 A2

(12) **EUROPEAN PATENT APPLICATION**

(21) Application number: **91118201.2**

(51) Int. Cl.[5]: **C07D 501/36**, A61K 31/545

(22) Date of filing: **25.10.91**

(30) Priority: **26.10.90 JP 290251/90**

(43) Date of publication of application:
**29.04.92 Bulletin 92/18**

(84) Designated Contracting States:
**AT BE CH DE DK ES FR GB GR IT LI LU NL SE**

(71) Applicant: **TAKEDA CHEMICAL INDUSTRIES, LTD.**
**1-1, Doshomachi 4-chome**
**Chuo-ku, OSAKA(JP)**

(72) Inventor: **Sendai, Michiyuki**
**1-1505, 37 Senriyamanishi 4-chome**
**Suita, Osaka 565(JP)**
Inventor: **Iwahi, Tomoyuki**
**B-407, 31 Yamadahigashi 1-chome**
**Suita, Osaka 565(JP)**
Inventor: **Tomimoto, Mitsumi**
**45-4, Daiwahigashi 5-chome**
**Kawanishi, Hyogo 666-01(JP)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **Cephem compounds, their production and use.**

(57) A novel compound of the formula:

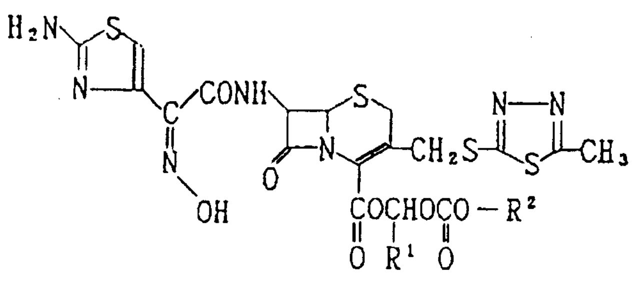

wherein $R^1$ stands for a $C_{1-3}$ alkyl group, $R^2$ stands for a $C_{3-5}$ alkyl group branched at $\alpha$-position or a $C_{3-5}$ cycloalkyl group, or a salt thereof, being useful as a therapeutic drug for bacterial infection.

EP 0 482 657 A2

## FIELD OF THE INVENTION

This invention relates to a novel cephem compound and a salt thereof for oral administration, which are useful as a chemotherapeutic agent.

## BACKGROUND OF THE INVENTION

While there are a number of cephem-type preparations having excellent antibacterial activity, most of them are injections and preparations for oral administration are relatively less in number. On the other hand, the antibacterial activity and spectrum of cephem-type preparations for oral administration are weaker than those of cephem-type preparations for injection. For solving this problem, extensive studies have been carried out for converting the carboxyl group at the 4-position of an injectable cephem compound or a cephem compound which is hardly absorbed by oral route to an ester which is capable of being de-esterified in vivo (e.g. cefuroxime axetil, cefotiam hexetil, cefpodoxime proxetil, cefteram pivoxil, etc.), which is a so-called "ester-type prodrug". However, no satisfactory compound has yet been obtained, from the viewpoints of antibacterial activity and antibacterial spectrum.

While it has been known that 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylic acid (hereinafter called "said cephem carboxylic acid") represented by the formula,

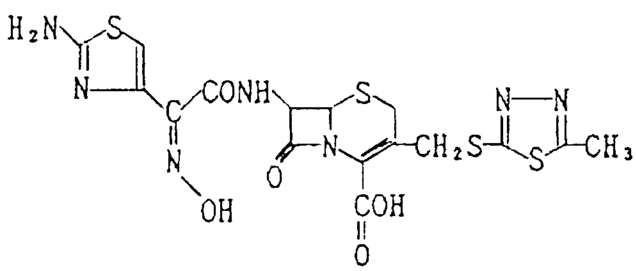

has an excellent antibacterial activity and a broad antibacterial spectrum, the said cephem carboxylic acid was not a compound which can be applied as it is for use in oral administration. And, while the pivaloyloxymethyl ester of the said cephem carboxylic acid and 1-(ethoxycarbonyloxy)ethyl ester of the said cephem carboxylic acid have been synthesized as ester-type prodrugs [Japanese Published Unexamined patent application No. 9698/1985], these esters are not satisfactory in respect of absorbability from intestinal tract. Under the circumstances, cephem agent for oral use, which have excellent antibacterial activity and broad antimicrobial spectrum and are improved in oral absorbability, have been desired.

## SUMMARY OF THE INVENTION

The present inventors have continued diligent study on cephem compounds for oral administration, and, as the result, they found that the ester compound obtained by a method characterized by selecting the said cephem carboxylic acid from an exceedingly large number of cephem compounds and by introducing into the carboxyl group at the 4-position of thus-selected cephem carboxylic acid the group represented by the formula:

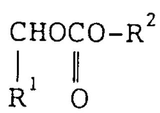

wherein $R^1$ stands for a $C_{1-3}$ alkyl group, $R^2$ stands for a $C_{3-5}$ alkyl group branched at the α-position or a $C_{3-5}$ cycloalkyl group, i.e. the cephem compound represented by the formula,

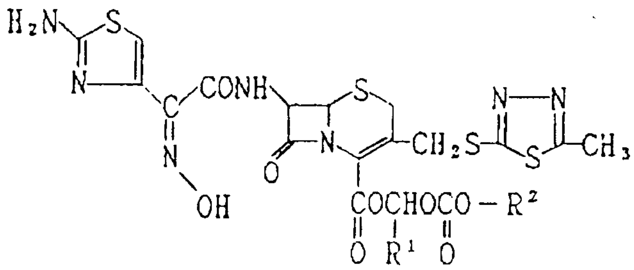

[ I ]

wherein the symbols are of the same meaning as defined above, or salts thereof have, unexpectedly, various excellent properties as cephem compounds for oral administration, as exemplified by

(i) good absorbability from intestinal tract,
(ii) high blood concentration of the said cephem carboxylic acid and long half-life thereof,
(iii) good urinary recovery rate of the said cephem carboxylic acid,
(iv) good protective activity by oral administration even against intraperitoneal infections by Staphylococcus aureus or Escherichia coli, and
(v) less side-effects and high safety.

And, as the characteristic features of the group constituting the ester bringing about these excellent properties, the following points are mentioned, i.e.

(1) $R^2$ is bonded via

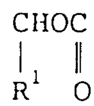

and O,
(2) $R^2$ stands for a $C_{3-5}$ alkyl group,
(3) the $C_{3-5}$ alkyl group is branched, and
(4) the branched alkyl group shown by $R^2$ (including $C_{3-5}$ cycloalkyl) is branched at the $\alpha$-position, and, based on the foregoing, the present invention has been accomplished.

## DETAILED DESCRIPTION OF THE INVENTION

In the above formulae, examples of $C_{1-3}$ alkyl groups shown by $R^1$ include methyl, ethyl and propyl, preferably methyl.

Examples of the $C_{3-5}$ alkyl group branched at its $\alpha$-position shown by $R^2$ include isopropyl, 1-methylpropyl and 1-ethylpropyl, preferably isopropyl.

Examples of the $C_{3-5}$ cycloalkyl group shown by $R^2$ include cyclopropyl, cyclobutyl and cyclopentyl, preferably cyclopentyl.

And, the compound [I] can be used as salts thereof. In this case, salts with acids can be used. Examples of such acids include mineral acids such as hydrochloric acid, sulfuric acid or phosphoric acid and organic acids such as maleic acid, acetic acid, citric acid, succinic acid, tartaric acid, malic acid, malonic acid, fumaric acid, benzoic acid, mandelic acid, ascorbic acid or methanesulfonic acid, which are known, in the fields of penicillin or cephalosporin, as acids forming pharmaceutically acceptable salts.

In the compound [I] or salts thereof, due to the existence of an asymmetric carbon at the ester moiety of the carboxyl group at the 4-position of the cephem skeleton, there are two kinds of optically active compounds (R-isomer, S-isomer) based thereon. Therefore, while the compound [I] or a salt thereof may usually be in the state of a diastereomer mixture having a suitable ratio, the R-isomer or the S-isomer isolated by a conventional method can be used.

The compound [I] or salts thereof are readily absorbed from the intestinal tract, and the ester moiety of the carboxyl group at the 4-position is promptly hydrolyzed by the enzyme in the body so that the compound [I] or salts thereof are converted to a non-ester compound of the compound [I], i.e. the said cephem carboxylic acid.

The compound [I] or salts thereof are valuable antibiotics showing excellent antibacterial activities against both gram-positive and gram-negative bacteria including those isolated clinically, which are used as medicines for humans and domestic animals and can be used safely as antibacterial agents for the therapy and prophylaxis of infections caused by various bacteria.

The compound [I] or salts thereof can be used, as therapeutic agents of bacterial infections, for the therapy and prophylaxis of, for example, respiratory infections, urinary tract infections, suppurative diseases, bile tract infections, intestinal infections, gyneco-obstetrical infections, otorhinological infections and surgical infections.

The compound [I] or salts thereof of the present invention can be orally administered, and they can be prepared into, for example, capsules, powders, triturations, granules and tablets by conventional methods after suitably mixing with per se known pharmaceutically acceptable excipients (e.g. starch, lactose, calcium carbonate or calcium phosphate), binders (e.g. starch, gum arabic, carboxymethyl cellulose, hydroxypropylcellulose or crystalline cellulose), lubricants (e.g. magnesium stearate or talc), disintegrators (e.g. carboxymethylcellulose calcium or talc), or the like.

And, a compound [I] or a salt thereof can be prepared into granules by a conventional method after incorporating a solid organic acid (e.g. citric acid, malic acid, tartaric acid, succinic acid, ascorbic acid and mandelic acid) in an amount of about 1 to 5 times as much as the compound [I] or its salt. These granules can be prepared into capsules, tablets, etc. by conventional methods.

The oral dosage of the compound [I] or a salt thereof per day for infectious diseases varies with the conditions, body weight, etc. of the patient, and it ranges from about 0.5 mg to 100 mg, preferably 1 mg to 20 mg, of the active component (the compound [I] or a salt thereof) per kg of body weight of an adult human, divided into 1 to 3, preferably 1 or 2 times.

The compound [I] or salts thereof can be produced by the methods set forth below.

Method 1

A compound [1] or a salt thereof can be produced by allowing a compound represented by the formula:

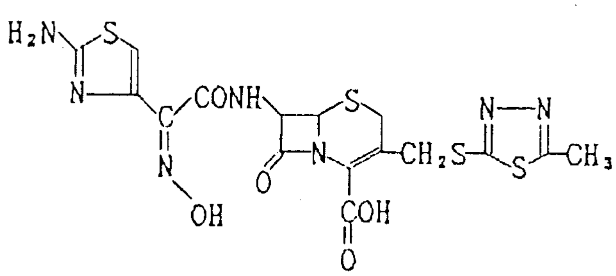

[II]

or a salt thereof to react with a compound represented by the formula:

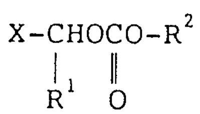

[III]

wherein X stands for a leaving group and $R^1$ and $R^2$ are of the same meaning as defined above.

In the above formula [III], as the leaving group shown by X, use is made of, for example, halogen such as chlorine, bromine or iodine, a $C_{6-10}$ aryl sulfonyloxy group such as phenylsulfonyloxy group or p-toluenesulfonyloxy group, or $C_{1-6}$ alkylsulfonyloxy group such as methylsulfonyloxy group. Among these, iodine is especially preferable as X.

And, since the compound [III] has an asymmetric carbon atom, it is subjected to optical resolution by a per se conventional means, then used for the reaction as the R-isomer or S-isomer or a mixture thereof.

On the other hand, the starting compound [II] may be subjected to the reaction as a salt with a base, for example, an alkali metal such as sodium or potassium, an alkaline earth metal such as calcium or magnesium, an organic amine such as triethylamine, trimethylamine, pyridine, colidine or lutidine.

Usually this reaction is conducted desirably in a solvent inert to the reaction. As the inert solvent, any one can be used so long as it does not hamper the reaction, and examples of suitable solvents include amides such as N,N-dimethylformamide (hereinafter abbreviated as "DMF") or N,N-dimethylacetamide (hereinafter abbreviated as "DMA"), halogenated hydrocarbons such as dichloromethane or chloroform, ethers such as dioxane or tetrahydrofuran (hereinafter abbreviated as "THF"), nitriles such as acetonitrile, sulfoxides such as dimethylsulfoxide (hereinafter abbreviated as "DMSO"), sulfolane, liquefied sulfur dioxide.

The compound [III] is used preferably in an amount of 1 to 5 mols. relative to 1 mol. of the compound [II] or a salt thereof. While the reaction temperature varies with various conditions, for example, the starting compounds and kinds of solvents, it ranges usually from -30°C to to 60°C, preferably -20°C to 30°C. While the reaction time varies with various conditions, for example, the starting compounds, kinds of solvents and reaction temperatures, it ranges usually from one minute to 24 hours, preferably 5 minutes to 5 hours.

The starting compound [II] which is used in this reaction can be synthesized by the method disclosed in GB-A-1587941 or a method analogous thereto. And, the starting compound [III] can be easily produced by a conventional method (e.g. EP-A-128029, Japanese Published Unexamined Patent Application No. 239490/1985) or a method analogous thereto.

Method 2

A compound [I] or a salt thereof can be produced by allowing a cephem compound [IV] represented by the formula:

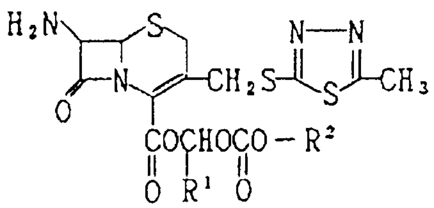

[IV]

wherein the symbols are of the same meaning as defined above, or a salt thereof to react with a compound of the formula:

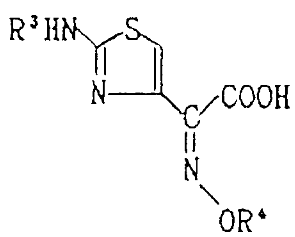

[V]

wherein $R^3$ stands for hydrogen atom or a protecting group of the amino group and $R^4$ stands for hydrogen atom or a protecting group of hydroxyl group, or a salt thereof or a reactive derivative at its carboxyl group, and, further, when necessary, by removing the protecting group or groups.

In the formula [V], as the amino-protecting group shown by $R^3$, use is suitably made of, for example, those employable in the fields of β-lactam and peptide, and, among them, chloroacetyl, tert-butoxycarbonyl, benzyloxycarbonyl, p-methoxybenzyloxycarbonyl, p-nitrobenzyloxycarbonyl, 2-trimethylsilylethoxycarbonyl, 2,2,2-trichloroethoxycarbonyl or trityl is preferable. Among them, chloroacetyl, tert-butoxycarbonyl or trityl is especially preferable.

As the hydroxy-protecting group shown by $R^4$, use is made of, for example, chloroacetyl, benzyl, p-nitrobenzyl, methylthiomethyl, trimethylsilyl, tert-butyldimethylsilyl, 2-tetrahydropyranyl, 4-methoxy-4-tetrahydropyranyl, or trityl. Among them, 2-tetrahydropyranyl, 4-methoxy-4-tetrahydropyranyl and trityl are especially preferable.

As salts of the compound [IV], use is made of, for example, salts with acids similar to those used in the case of the above-mentioned salts of the compound [I]. As salts of the compound [V], salts similar to those described in the above-mentioned [II].

As reactive derivatives at the carboxyl group of the compound [V], use is made of, for example, acid halides, acid anhydrides, active amides, active esters, active thioesters, etc., which are more specifically described below.

1) Acid halides:

For example, acid chlorides, acid bromides etc. are employed.

2) Acid anhydrides:

For example, mono-lower alkylcarbonic acid mixed anhydride are used.

3) Active amides:

For example, amides formed with pyrazole, imidazole, 4-substituted imidazole, dimethylpyrazole, benzotriazole, etc. are used.

4) Active ester:

For example, esters such as methoxymethyl esters, benzotriazole esters, 4-nitrophenyl esters, 2,4-dinitrophenyl esters, trichlorophenyl esters, pentachlorophenyl esters, etc., as well as esters formed with 1-hydroxy-1H-2-pyridone, N-hydroxysuccinimide, N-hydroxyphthalimide or the like.

5) Active thioesters:

For example, thioesters formed with, for example, heterocyclicthiols such as 2-pyridylthiol, 2-benzothiazolylthiol, etc. are used.

In this reaction, the compound [V] or a salt thereof or a reactive derivative at its carboxyl group is used in an amount of one mol or more, preferably in a range of from about one mol. to 4 mols. relative to 1 mol. of the compound [IV] or a salt thereof. This reaction may be conducted usually in a solvent which is exemplified by water, ketones such as acetone, etc., ethers such as THF, dioxane, etc., nitriles such as acetonitrile, etc., halogenated hydrocarbons such as dichloromethane, chloroform, 1,2-dichloroethane, etc., esters such as ethyl acetate, etc., amides such as DMF, DMA, etc. These solvents may be used singly or in combination of two or more of them in a suitable mixture ratio. When the compound [V] is used as the free acid or as a salt thereof, the reaction may be preferably carried out in the presence of a condensing agent which is exemplified by N,N-dicyclohexylcarbodiimide, N-cyclohexyl-N-morpholinoethylcarbodiimide, N-cyclohexyl-N-(4-diethylaminocyclohexyl)carbodiimide, N-ethyl-N-(3-dimethylaminopropyl)carbodiimide, etc. The reaction can also be carried out in the presence of a base which includes, for example, alkali metal carbonates such as sodium carbonate, potassium carbonate, sodium hydrogencarbonate, potassium hydrogencarbonate, etc., tertiary amines such as triethylamine, tributylamine, N-methylmorpholine, N-methylpiperidine, etc., and aromatic amines such as pyridine, picoline, lutidine, colidine, N,N-dimethylaniline, etc. These bases serve to accelerate the reaction, to neutralize the acid formed during the reaction or to make the starting materials to be readily soluble. The amount of such a base may usually be about 0.01 to 5 mols., preferably about 0.1 to 2 mols. relative to 1 mol. of the compound [V] or a salt thereof.

While the reaction temperature varies with reaction conditions including the starting materials and kinds of the solvents then employed, it ranges from -50 °C to 100 °C, preferably -20 °C to 50 °C. While the reaction time varies with reaction conditions including the starting materials, kinds of the solvents, reaction temperatures, etc., it ranges from one minute to 24 hours, preferably five minutes to 10 hours.

The starting compound [IV] to be used for this reaction can be synthesized in accordance with, for example, the method described in Japanese Published Unexamined Patent Application No. 239490/1985. And, the starting compound [V] can be synthesized by, for example, the method described in GB-A-1580621.

Method 3

A compound [I] or a salt thereof can be produced by allowing a starting compound represented by the formula:

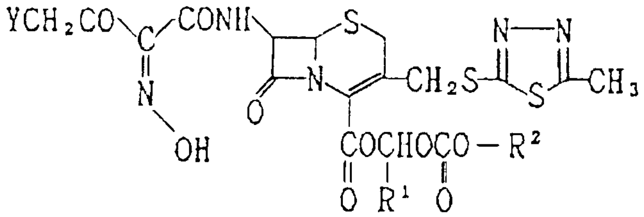

[VI]

wherein Y stands for halogen atom, and the other symbols are of the same meaning as defined above, to react with thiourea in an inert solvent.

As the halogen atom of Y, use may be made of, for example, chlorine, bromine or iodine, preferably, for example, chlorine or bromine.

Cyclization of the compound [VI] with thiourea can be carried out in accordance with a per se known method (e.g. the method disclosed in, for example, GB-A-1587941).

While the reaction temperature varies with various conditions including types of the starting compounds and kinds of the solvents employed, it ranges from -20 ° C to 50 ° C, preferably 0 ° C to 30 ° C. While the reaction time varies with various conditions including types of the starting compounds, kinds of the solvents and reaction temperatures employed, it ranges from 5 minutes to 24 hours, preferably 10 minutes to 5 hours. The reaction is preferably conducted in an inert solvent. As the inert solvent, while any one may be used so long as it does not hamper the reaction, such amides, ketones and acetonitriles as described in the above Method 2 are preferable.

The starting compound [VI] can be produced by allowing diketene to react with halogen (especially chlorine and bromine are preferable) in accordance with a per se known method (e.g. the method disclosed in British Patent No. 1587941), by allowing the resultant product to react with a compound [IV] or a salt thereof to give a compound represented by the formula:

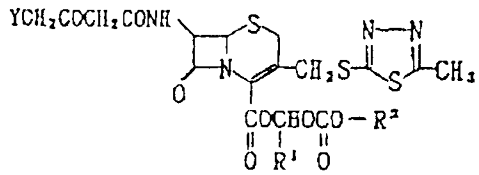

[VII]

wherein the symbols are of the same meaning as defined above, then by subjecting the compound [VII] to nitrosation.

When protecting groups are contained in the reaction products of the above-mentioned Methods 1 to 3, the compound [I] or a salt thereof can be obtained by, when necessary, removing the protective group or groups by a conventional means.

The object compound [I] or its salt thus obtained can be isolated and purified by a conventional method, for example, solvent-extraction, pH-change, phase transfer, salting out, crystallization, recrystallization, chromatography, etc.

In the above-mentioned Methods 1 to 3, the compound [I] (syn[Z]-isomer) or a salt thereof is obtained sometimes as a mixture with its anti[E]isomer. For isolating the desired syn-isomer (i.e. the compound [I] or a salt thereof) from the mixture, a per se conventional method or a method analogous thereto can be applied, as exemplified by separation utilizing the difference in solubility, crystallizability, etc. or isolation by means of chromatography, etc.

The compound [I] or salts thereof obtained by the present invention have excellent properties as cephem compounds for oral administration. As concrete examples of those properties, therapeutic effects against intraperitoneal infections in mice by oral administration, and the urinary recovery rate of the said

cephem carboxylic acid and effects of maintaining the concentration of the said cephem carboxylic acid in blood after oral administration of the compound [I] in mice are described as follows in comparison with control compounds.

(1) Therapeutic Effects against Intraperitoneal

Infections in Mice. Test animals: Slc : ICR, 4-week old male mice (body weight 20 to 22 g) were employed. Induction of experimental infections (infected mice):

Staphylococcus aureus 308A-1 or Escherichia coli 0-111 was cultured in Brain Heart Infusion broth (manufactured by Difco Co.) at 37° C for 20 hours. Each of the organisms was suspended in a 8% mucin solution (manufactured by Difco in) at a concentration of 2 x $10^8$ Colony Forming Units (hereinafter abbreviated as "CFU")/ml and 2 x $10^5$ CFU/ml, respectively, and 0.5 ml of each suspension was administered intraperitoneally. Administration of Test compounds: 25 mg of each test compound was dissolved in 0.5 ml of DMSO, and each solution was diluted in distilled water to prepare two fold dilutions of each compound in the range of 2.5 mg/ml - 0.156 mg/ml in the case of infection with Staphylococcus aureus and in the range of 0.156 mg/ml - 0.01 mg/ml in the case of infection with Escherichia coli. A volume of 0.2 ml of each dilution was once administered orally using a tube into the stomach of five mice each in each group immediately after the infection.

Determination of the effect: Thus-treated mice were observed for five days, and the survival rates with the respective amounts of administration were counted, then $ED_{50}$(mg/kg) was calculated by the method of Reed and Münch. The $ED_{50}$ value of each compound was that in terms of the potency of the said cephem carboxylic acid. The results are shown in the following table.

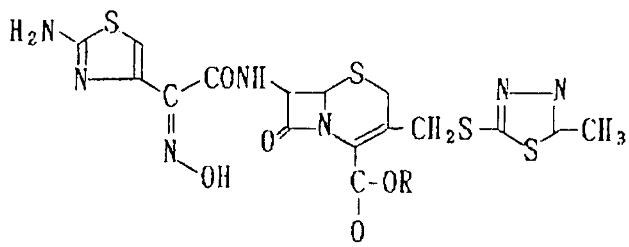

Organism : <u>Staphylococcus</u> <u>aureus</u> 308A－1

| Test Compound | | R | $ED_{50}$ (mg/kg) |
|---|---|---|---|
| The Compound of this Invention | Ex. 1 | $-CHOCOOCH(CH_3)_2$<br>\|<br>$CH_3$ | 7.02 |
| | Ex. 2 | $-CHOCOOCH(CH_3)_2$<br>\|<br>$CH_3$<br>(HCl salt) | 5.57 |
| | Ex. 3 | -CHOCOO— 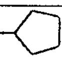<br>\|<br>$CH_3$ | 8.84 |
| | Ex. 5 | -CHOCOO— 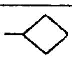<br>\|<br>$CH_3$<br>(HCl salt) | 14.0 |
| | Ex. 6 | $-CHOCOOCH(C_2H_5)_2$<br>\|<br>$CH_3$<br>(HCl salt) | 12.5 |

| Test Compound | | R | $ED_{50}$ (mg/kg). |
|---|---|---|---|
| The known Compound | GB-A-1587941 Ex.16 | -H (Na salt) | > 25.0 |
| | JP-A-9698/1985 PR Ex.3 | $-CHOCOC(CH_3)_3$ <br> \| <br> H | 22.3 |
| | JP-A-9698/1985 PR Ex.4 | $-CHOCOOC_2H_5$ <br> \| <br> $CH_3$ | > 25.0 |
| Novel analogous compound | | 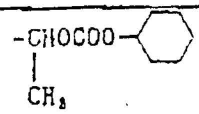 | > 25.0 |
| | | $-CHOCOCH_3$ <br> \| <br> H <br> (HCl salt) | > 25.0 |
| | | 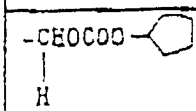 <br> (HCl salt) | 17.7 |
| | | 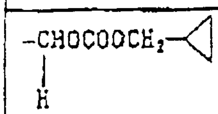 <br> (HCl salt) | > 25.0 |

(JP-A- : Japanese published unexamined Patent Application No.

PR EX.: Preparation Example )

Organism : Escherichia coli O-111

| Test Compound | | R | $ED_{50}$ (mg/kg) |
|---|---|---|---|
| The Comound of this invention | Ex. 1 | $-CHOCOOCH(CH_3)_2$<br>\|<br>$CH_3$ | 0.141 |
| | Ex. 2 | $-CHOCOOCH(CH_3)_2$<br>\|<br>$CH_3$<br>(HCl salt) | 0.130 |
| | Ex. 3 | 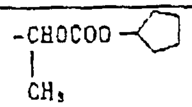 | 0.130 |
| The Known Compound | GB-A-1587941 Ex.16 | -H<br>(Na salt) | 1.25 |
| | JP-A-9698/1985 PR Ex.3 | $-CHOCOC(CH_3)_3$<br>\|<br>H | 0.279 |
| | JP-A-9698/1985 PR Ex.4 | $-CHOCOOC_2H_5$<br>\|<br>$CH_3$ | 0.349 |
| Novel analogous compound | | $-CHOCOCH_3$<br>\|<br>H<br>(HCl salt) | >0.780 |
| | | 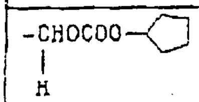<br>(HCl salt) | 0.552 |
| | | 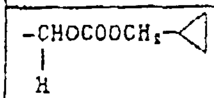<br>(HCl salt) | 0.435 |

(JP-A- : Japanese published unexamined Patent Application No.

PR EX.: Preparation Example )

This test shows that the compound [I] or salts thereof have excellent therapeutic effects, compared with the known compounds and the novel analogous compounds.

(2) Urinary Recovery rate in mice

Test animals : Slc : ICR, 4-week old male mice (20 - 22 g body weight) were employed.
Dosage : 10mg of each test compound was dissolved in 0.5 ml of DMSO. The respective solutions were diluted 10 times with 50%(v/v) of aqueous solution of polyethyleneglycol 200. A volume of 0.1 ml/10 g body

weight of each of the respective dilutions was administered once to normal mice (five in one group) orally into stomach by using a tube.

Recovery of urine: After administration of the drug, each test animal was placed separately in a cage, and urine was collected during 24 hours.

Determination of drug concentration: The concentration of the said cephem carboxylic acid in urine was determined by the agar well method using, as test bacteria, Escherichia coli NIHJ and Proteus rettgeri ATCC 9250.

The results are set forth as follows.

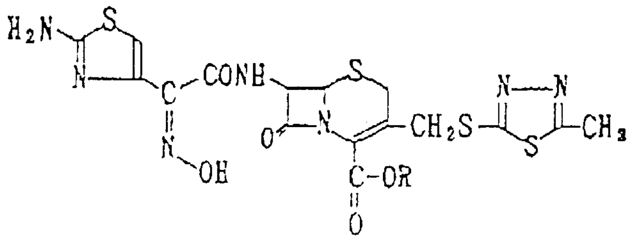

| Test Compound | | R | Urinary excretion(%) |
|---|---|---|---|
| The Compound of this Invention | Ex. 1 | $-CHOCOOCH(CH_3)_2$<br>\|<br>$CH_3$ | 16.2 |
| | Ex. 2 | $-CHOCOOCH(CH_3)_2$<br>\|<br>$CH_3$<br>(HCl salt) | 25.7 |
| | Ex. 3 | 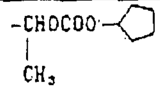 | 25.5 |
| | Ex. 5 | 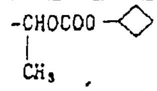<br>(HCl salt) | 21.1 |
| The Known Compound | GB-A-1587941 Ex. 16 | -H<br>(Na salt) | 2.11 |
| | JP-A-9698/1985 PR Ex. 3 | $-CHOCOC(CH_3)_3$<br>\|<br>H | 13.8 |
| | JP-A-9698/1985 PR Ex. 4 | $-CHOCOOC_2H_5$<br>\|<br>$CH_3$ | 11.6 |
| Novel analogous Compound | | 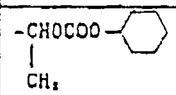<br>(HCl salt) | 7.61 |
| | | $-CHOCOCH_3$<br>\|<br>H<br>(HCl salt) | 6.0 |
| | | 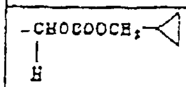<br>(HCl salt) | 6.8 |

(JP-A- : Japanese published unexamined Patent Application No.

PR EX.: Preparation Example )

This test result shows that the compound [I] has a high urinary recovery rate, compared with the known compounds and the novel analogous compounds.

(3) Blood concentration of the said cephem carboxylic acid

Test animals: Slc : ICR, 4-week old male mice (20 - 22 g body weight) were employed.

Dosage : 10 mg of each test compound was dissolved in 0.5 ml of DMSO. The respective solutions were diluted 10 times with 50% (v/v) of aqueous solution of polyethylene glycol 200. 0.1 ml/10 g body weight of each of the respective dilution solutions was administered once to normal mice (five in one group) orally

into stomach by using a tube.

Recovery of test materials: Blood was collected in a given period after administration of drugs.

Determination of concentration of drug: The blood concentration of the said cephem carboxylic acid was determined by the agar well method using, as test bacteria, Escherichia coli NIHJ and Proteus rettgeri ATCC 9250.

The results are set forth as follows.

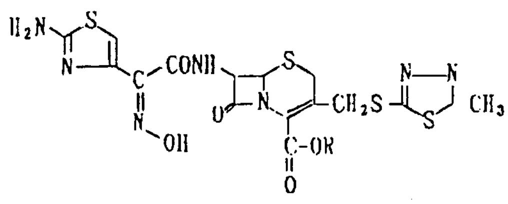

| | | | Concentration in blood (μg/ml) | | | | | |
|---|---|---|---|---|---|---|---|---|
| Test Compound | | Hour<br>R | 0.25 | 0.5 | 1 | 2 | 4 | AUC<br>μg.h/ml |
| The Compound of this invention | | $-CHOCOOCH(CH_3)_2$<br>\|<br>$CH_3$ | 35.5 | 34.9 | 25.5 | 10.2 | 2.99 | 58.4 |
| The Known | JP-A-9698/1985 PR Ex.3 | $-CHOCO(CH_3)_3$<br>\|<br>H | 22.0 | 19.4 | 15.4 | 6.4 | 2.41 | 38.7 |
| Compound | JP-A-9698/1985 PR Ex.4 | $-CHOCOOC_2H_5$<br>\|<br>$CH_3$ | 15.8 | 28.8 | 19.0 | 6.4 | 2.56 | 40.3 |

(JP-A- : Japanese published unexamined Patent Application No.

PR EX.: Preparation Example )

This test result shows that administration of the compound [I] or salts thereof serve to maintain high blood concentration of the said cephem carboxylic acid for a long period of time, compared with the known compounds.

## Examples

By the following working examples, the present invention will be described in further detail. However, these are nothing more than mere examples and not intended to limit the scope of this invention in any manner.

In the following working examples, NMR spectrum was measured by means of Gemini 200 (200MHz)-spectrometer, and all the δ values were shown by ppm. Symbols in the examples have the following meanings.

| | |
|---|---|
| s | : singlet |
| d | : doublet |
| t | : triplet |
| ABq | : AB type quartet |
| dd | : double doublet |
| m | : multiplet |
| br. | : broad |
| J | : coupling constant |
| sh | : shoulder |

Example 1

1-(Isopropoxycarbonyloxy)ethyl 7β-[2-(2-aminothiazol-4-yl)-(Z)-2-hydroxyiminoacetamido ]-3-[(2-methyl-1,3,4-thiazol-5-yl)thiomethyl]-3-cephem-4-carboxylate

In DMA (3.5 ml) was dissolved sodium 7β-[2-(2-aminothiazol-4-yl)-(Z)-2-hydroxyiminoacetamido]-3-[(2-methyl-1-3,4-thiazol-5-yl)thiomethyl]3-cephem-4-carboxylate (0.2 g). To the solution was added, with stirring under ice-cooling, 1-(isopropoxycarbonyloxy)ethyliodide (0.3 g). The mixture was stirred for 30 minutes at the same temperature, and there was added ice-water, followed by extraction with ethyl acetate. The organic layer was washed with brine, dried over anhydrous sodium sulfate, then the solvent was distilled off under reduced pressure. The residue was dissolved in acetone (3 ml), to which was added isopropyl ether (30 ml) with stirring at room temperature (20 ° C). Precipitates were collected by filtration and washed with isopropyl ether, followed by drying to afford 135 mg of the above-titled compound as a mixture of two diastereomers (about 1:1) as a pale yellow powder.

IR(KBr)$cm^{-1}$: 3325, 3200, 2980, 1780, 1755, 1670, 1610, 1530

$^1$H-NMR(DMSO-$d_6$) δ: 1.20-1.25(6H,m), 1.50 and 1.53(3H, each d,J = 5Hz), 2.68 and 2.69(3H, each s), 3.71-(2H,ABq,J = 18Hz), 4.34 and 4.36(2H, each ABq,J = 14Hz), 4.71-4.86(1H,m), 5.16 and 5.19(1H, each d, J = 5Hz), 5.79-5.88(1H,m), 6.66(1H,s), 6.77-6.92(1H,m), 7.25(2H,brs), 9.48 and 9.49(1H, each d, J = 8Hz)

Example 2

1-(Isopropoxycarbonyloxy)ethyl 7β-[2-(2-aminothiazol-4-yl)-(Z)-2-hydroxyiminoacetamido ]-3-[(2-methyl-1,3,4-thiadiazol-5-yl)thiomethyl]-3-cephem-4-carboxylate hydrochloride

In ethyl acetate (15 ml) was dissolved the compound (100 mg) obtained in Example 1, to which was added, with stirring under ice-cooling, 0.4N hydrochloric acid solution in ethyl acetate (0.5 ml). Powdery precipitates were collected by filtration, washed with ethyl acetate, and dried under reduced pressure to afford 78 mg of the above-titled compound as a mixture of two diastereomers (about 1:1) as a pale yellow powder.

IR(KBr)$cm^{-1}$: 2980, 1780, 1750, 1670, 1620, 1520

$^1$H-NMR(DMSO-$d_6$) δ: 1.22-1.26(6H,m), 1.51 and 1.53(3H, each d,J = 5Hz), 2.69(3H,s), 3.59-3.89(2H,m), 4.34 and 4.40(2H, each ABq,J = 13Hz), 4.72-4.87(1H,m), 5.21 and 5.23(1H, each d,J = 5Hz), 5.79-5.89(1H,m), 6.81 and 6.88(1H, each q,J = 5Hz), 6.85(1H,s), 9.72 and 9.73 (1H, each d, J = 8Hz)

Example 3

1-(Cyclopentyloxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate

In the same procedure as in Example 1, using 1-(cyclopentyloxycarbonyloxy)ethyliodide in place of 1-(isopropoxycarbonyloxy)ethyliodide the above-titled compound was obtained as a mixture of two diastereomers (about 1:1).

IR(KBr)$cm^{-1}$: 3325, 3190, 2970, 2875, 1780, 1755, 1675, 1610, 1530

$^1$H-NMR(DMSO-$d_6$) δ: 1.4-1.9(11H,m), 2.69(3H,s), 3.55-4.60(4H,m), 4.96-5.07(1H,m), 5.17 and 5.19(1H, each d,J = 5Hz), 5.80-5.90(1H,m), 6.66(1H,s), 6.80 and 6.88(1H, each q, J = 5Hz), 7.21(2H,brs), 9.48 and 9.49(1H, each d,J = 8Hz)

Example 4

1-(Cyclopentyloxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride

Using the compound obtained in Example 3, a reaction was allowed to proceed in the same procedure as that in Example 2 to afford the above-titled compound as a mixture of two diastereomers (about 1:1).

IR(kBr)$cm^{-1}$: 2970, 1785, 1750, 1675, 1620, 1520

$^1$H-NMR(DMSO-$d_6$)δ: 1.45-1.90(11H,m), 2.69(3H,s), 3.74 and 3.75(2H, each ABq, J = 18Hz), 4.33 and 4.39-(2H, each ABq, J = 13Hz), 4.96-5.06(1H,m), 5.78-5.88(1H,m), 6.77-6.91(1H,m), 6.84(1H,s), 9.05(2H,brs), 9.73-(1H,d,J = 8Hz)

Example 5

1-(Cyclobutyloxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride

Using 1-(Cyclobutyloxycarbonyloxy)ethyliodide in place of 1-(isopropoxycarbonyloxy)ethyliodide in Example 1, a reaction was allowed to proceed in the same procedure as that in Example 1 and Example 2 to afford the above titled compound as a mixture of two diastereomers (about 1:1).
IR(KBr)$cm^{-1}$: 2990, 1780, 1760, 1675, 1625, 1525
$^1$H-NMR(DMSO-$d_6$)δ: 1.5-2.4(6H,m), 1.51 and 1.53(3H, each d, J = 5Hz), 2.69(3H,s), 3.74 and 3.75(2H, each ABq, J = 18Hz), 4.33 and 4.40(2H, each ABq, J = 14Hz), 4.75-4.96(1H,m), 5.20 and 5.22(1H, each d, J = 5Hz), 5.78-5.88(1H,m), 6.75-6.91(1H,m), 6.85(1H,s), 9.72 and 9.73(1H, each d, J = 8Hz)

Example 6

1-(1-Ethylpropoxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride

Using 1-(1-ethylpropoxycarbonyloxy)ethyliodide in place of 1-(isopropoxycarbonyloxy)ethyliodide in Example 1, a reaction was allowed to proceed in the same procedure as that in Example 1 and Example 2 to afford the above titled compound as a mixture of two diastereomers (about 1:1).
1R(KBr)$cm^{-1}$: 2975, 1780, 1750, 1675, 1620, 1525
$^1$H-NMR(DMSO-$d_6$)δ: 0.78-0.89(6H,m), 1.45-1.65(7H,m), 2.69(3H,s), 3.73 and 3.75(2H, each ABq, J = 18Hz), 4.14-4.60(3H,m), 5.21 and 5.23(1H,each d,J = 5Hz), 5.78-5.88(1H,m), 6.80 and 6.88(1H, each q, J = 6Hz), 6.84 and 6.85(1H,each s), 9.72 and 9.74(1H,each d, J = 8Hz)

Reference Example 1

Using 1-(cyclohexyloxycarbonyloxy)ethyliodide instead of 1-(isopropoxycaronyloxy)ethyliodide, a reaction was allowed to proceed in a manner similar to that of Example 1 to afford 1-(cyclohexyloxycarbonyloxy)ethyl 7β-[2-(2-aminothiazol-4-yl)-(Z)-2-hydroxyiminoacetamido]-3-[(2-methyl)-1,3,4-thiadiazol-5-yl)thiomethyl]-3-cephem-4-carboxylate as a mixture of two diastereomers (about 1:1).
IR(KBr)$cm^{-1}$: 3325, 2930, 2860, 1780, 1755, 1675, 1620, 1520
$^1$H-NMR(DMSO-$d_6$)δ: 1.10-1.94(13H,m), 2.69(3H,s), 4.13-4.65(3H,m), 5.17 and 5.20(1H,each d,J = 5Hz), 5.79-5.88(1H;m), 6.70 and 6.71 (1H,each s), 6.81 and 6.18(1H,each q,J = 6Hz), 7.2-7.7(2H,br), 9.54 and 9.55-(1H,each d,J = 8Hz)

## Claims

1. A cephem compound of the formula:

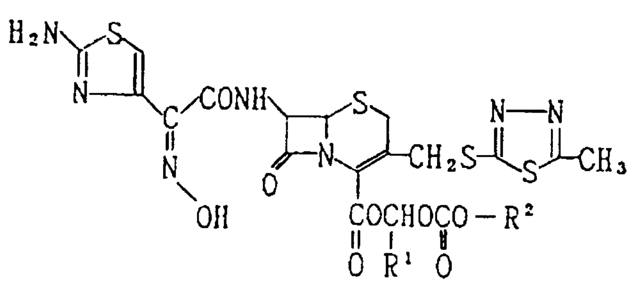

wherein $R^1$ stands for a $C_{1-3}$ alkyl group and $R^2$ stands for a $C_{3-5}$ alkyl group branched at the α-position or a $C_{3-5}$ cycloalkyl group, or a salt thereof.

2. A compound as claimed in Claim 1, wherein $R^1$ stands for methyl.

3. A compound as claimed in Claim 1, wherein $R^1$ stands for methyl and $R^2$ stands for a $C_{3-5}$ alkyl group branched at the α-position.

**4.** A compound as claimed in Claim 1, wherein $R^2$ is an isopropyl group.

**5.** A compound as claimed in Claim 1, wherein $R^2$ is a cyclopentyl group.

**6.** A compound as claimed in Claim 1, wherein the salt is a mineral acid salt.

**7.** A compound as claimed in Claim 1, the salt is a hydrochloric acid salt.

**8.** A compound as claimed in Claim 1 which is 1-(isopropoxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate.

**9.** A compound as claimed in Claim 1 which is 1-(cyclopentyloxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate.

**10.** A compound as claimed in Claim 1 which is 1-(cyclobutyloxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride.

**11.** A compound as claimed in Claim 1 which is 1-(1-ethylpropoxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride.

**12.** A compound as claimed in Claim 1, which is 1-(isopropoxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride.

**13.** A compound as claimed in Claim 1, which is 1-(cyclopentyloxycarbonyloxy)ethyl 7β-[2-(2-aminothiazol-4-yl)-(Z)-2-hydroxyiminoacetamido]-3-[(2-methyl-1,3,4-thiadiazol-5-yl)thiomethyl]-3-cephem-4-carboxylate hydrochloride.

**14.** An antibacterial agent which comprises an effective amount of a cephem compound of the formula:

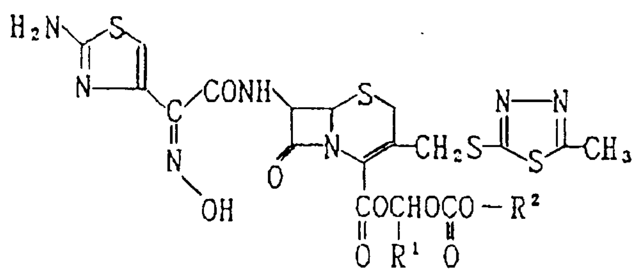

wherein the symbols are of the same meaning as defined in Claim 1, or a pharmaceutically acceptable salt thereof.

**15.** A method of producing a cephem compound of the formula:

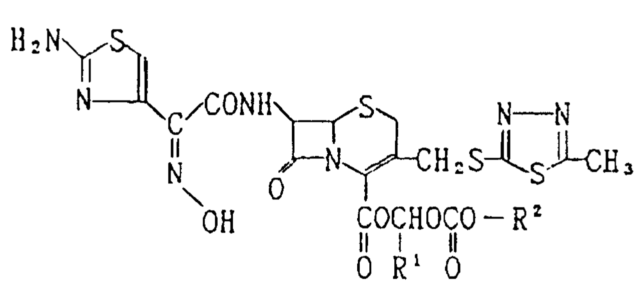

wherein the symbols are of the same meaning as defined in Claim 1, or a salt thereof, which comprises reacting a compound of the formula:

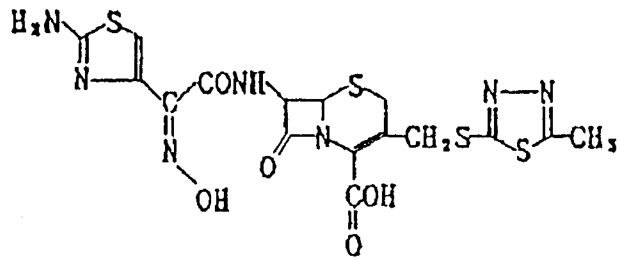

, or a salt thereof with a compound of the formula:

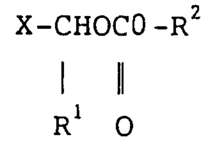

wherein X stands for a leaving group and $R^1$ and $R^2$ are of the same meaning as defined in Claim 1.

**16.** A method of producing a cephem compound of the formula:

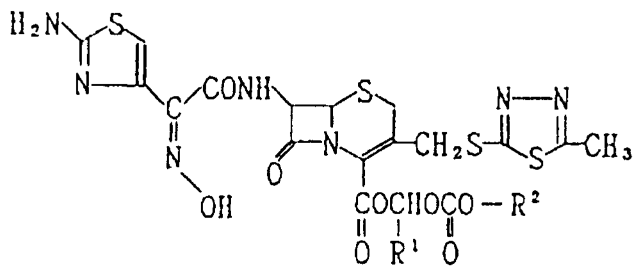

wherein the symbols are of the same meaning as defined in Claim 1, or a salt thereof, which comprises reacting a compound of the formula:

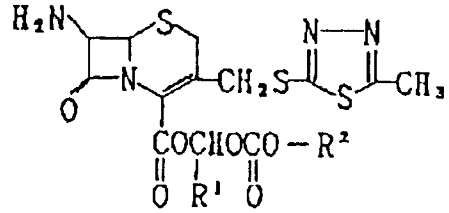

wherein the symbols are of the same meaning as defined in Claim 1, or a salt thereof with a compound of the formula:

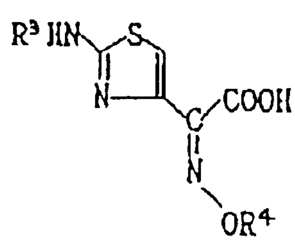

wherein $R^3$ stands for hydrogen atom or a protecting group of the amino group and $R^4$ stands for hydrogen atom or a protecting group of hydroxyl group, or a salt thereof or a reactive derivative at its carboxyl group, and if when necessary, by removing the protecting group or groups.

**17.** A method of producing a cephem compound of the formula:

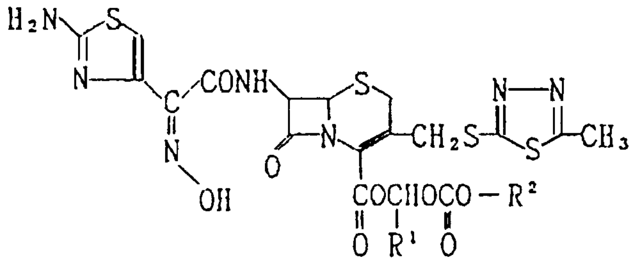

wherein the symbols are of the same meaning as defined in Claim 1, or a salt thereof, which comprises reacting a compound of the formula:

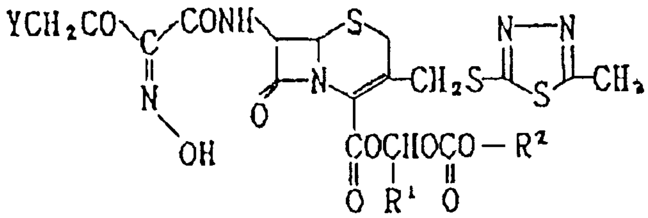

wherein Y stands for halogen atom, and the other symbols are of the same meaning as defined in Claim 1, with thiourea.

**18.** Use of a compound as claimed in Claim 1 as starting material in the preparation of an antibacterial agent.

**19.** A method for treating bacterial infection which comprises administering an effective amount of a compound as claimed in Claim 1 or a pharmaceutically acceptable salt thereof to a mammal in need thereof.

**20.** A method for maintaining antibacterial activity in blood for a long period of time which comprises administering an antibacterially effective amount of a compound as claimed in Claim 1 or a pharmaceutically acceptable salt thereof to a mammal suffering from bacterial infection.